# EUROPEAN PATENT APPLICATION

(11) **EP 1 112 750 A1**
(43) Date of publication of application: **04.07.2001**
(21) Application number: 00946272.2
(22) Date of filing: 12.07.2000
(51) Int. Cl.: A61K 47/32, A61K 47/38, A61K 47/44, A61K 47/16, A61K 47/14, A61K 45/00, A61P 1/02

(54) **DRUG COMPOSITION FOR TOPICAL ADMINISTRATION**

(30) Priority: 12.07.1999 JP 19801299
(71) Applicant: SUNTORY LIMITED, Osaka-shi, Osaka 530-8203 (JP); Sato Pharmaceutical Co. Ltd., Tokyo 107-0051 (JP)
(72) Inventor: TATARA, Mitsutoshi, Yokohama-shi, Kanagawa 240-0024 (JP); SHIMIZU, Toshihito, Adachi-ku, Tokyo 123-0845 (JP); FUKUMOTO, Ryoichi, Hachioji-shi, Tokyo 193-0816 (JP); NOMURA, Masaaki, Ohra-gun, Gunma 370-0708 (JP)
(74) Representative: HOFFMANN - EITLE
(86) International application number: JP0004651
(87) International publication number: WO0103742

(57) **Abstract**

A pharmaceutical composition for topical administration, which resides at a topical site of application for a long period and can control the release rate of an active agent, is provided. The present invention is a pharmaceutical composition for topical administration comprising an active ingredient, a water insoluble polymer, and a solvent capable dissolving the water insoluble polymer and compatible with water, and optionally containing other additive.

## Description

### TECHNICAL FIELD OF THE INVENTION

The present invention relates to a technique for topical administration of an active ingredient. More specifically, the invention relates to a pharmaceutical base which takes a soft gelled form suitable for administration before being applied topically, and which hardens after application and can hold an active ingredient topically for a long period; and a pharmaceutical composition for topical administration which has been prepared with the use of the base so as to achieve controlled release of a drug.

### PRIOR ART

Ointments, gels and creams, which can be administered easily and which are convenient in view of portability, are used as preparations for topical administration in various diseases. Ointments of antibiotics are useful particularly for topical inflammatory diseases due to infection with microorganisms, or pyogenic or infectious diseases. Therapeutic agents consisting of various antibiotics as an active ingredient have been demanded, and some of them find practical use.

As ointments consisting of various antibiotics as an active ingredient, for example, antibiotics, such as aminoglycosides, tetracyclines, and chloramphenicols, are frequently used in the treatment of inflammatory diseases and pyogenic diseases in the fields of dermatology, ophthalmology, otorhinolaryngology, dentistry/oral surgery, urology, and gynecology. Concretely, an ointment of kanamycin sulfate, which is an aminoglycoside antibiotic, an ointment of tetracycline hydrochloride, a tetracycline antibiotic, and an ointment of chloramphenicol, a chloramphenicol antibiotic, are commercially available as agents for treatment of pyogenic disease in dermatology. An ophthalmic ointment of a pimaricin preparation, which is a macrolide antibiotic, is also on the market. An ointment containing tetracycline hydrochloride, which is a tetracycline antibiotic, and hydrocortisone acetate, is sold for dentistry and oral surgery.

An ointment requires that its active ingredient be stably incorporated into a pharmaceutical composition. Japanese Patent Publication No. 12728/1989 describes that a pharmaceutical composition for topical administration, which has a magnesium compound incorporated into a hydrogel composed of a water-soluble polymer, a polyhydric alcohol and minocycline, one of tetracyclines, or its pharmaceutically acceptable salt, to stabilize the antibiotic, is used as a therapeutic agent administered topically for periodontal disease.

When an ointment is used as an intraoral preparation, for example, for treatment of periodontal disease, it is also necessary that its active ingredient be stable, and outflow of the active ingredient by saliva, effusion or blood be suppressed maximally. Japanese Patent Publication No. 34325/1990 improves the technique of Japanese Patent Publication No. 12728/1989, and describes that a composition, which comprises a suitable combination of a water soluble polymer (e.g., hydroxyethylcellulose), a certain type of methacrylate copolymer (e.g., aminoalkyl methacrylate copolymer RS), and a solubilizer therefor (e.g., triacetin) in addition to the combination of a magnesium compound (e.g., magnesium chloride (hexahydrate))-polyhydric alcohol (e.g., glycerin)-minocycline, resides at the site of administration for a long time, with a persistent effect, without impairing the stability of minocycline. Japanese Unexamined Patent Publication No. 89874/1995 describes a sustained release oral ointment characterized by containing a hydrophobic ointment base (e.g., plastibase), a polyhydric alcohol (e.g., propylene glycol), an adhesive substance (e.g., hydroxypropyl methylcellulose), a metal compound containing aluminum as a constituent (e.g., aluminum hydroxide), and an active ingredient. This ointment can be administered to a periodontal pocket, and is intended to increase the availability of the active ingredient while maintaining a sufficient concentration of the active ingredient at the lesion for a long period.

However, the development of a pharmaceutical manufacturing technology for retaining the active ingredient at a topical site, such as a periodontal pocket, stably and for a long period has just begun to take shape, and the development of better techniques is demanded strongly.

### SUMMARY OF THE INVENTION

In view of these circumstances, the inventors of the present application have gained the idea that imparting post-application shape retention properties to a composition is necessary to release an active ingredient stably for a long period. Based on this idea, they conducted extensive studies on pharmaceutical manufacturing techniques, and developed a hardening preparation for topical administration, thereby accomplishing the present invention.

The present invention provides a pharmaceutical base, especially a pharmaceutical base for topical administration, typically a pharmaceutical base for intraoral administration; which has at least a surface portion thereof hardening at a topical site of application, thereby residing at the topical site for a long period, to enable the effect of an active ingredient to persist.

The present invention also provides a preparation for topical administration, especially a preparation for intraoral administration, which comprises an active ingredient incorporated into the above base, and which has at least a surface portion thereof hardening after application to a topical site, thereby enabling it to reside at the topical site for a long period, to enable the effect of an active ingredient to persist for a long period.

The present invention further provides an ointment for intraoral administration, which comprises an active ingredient for treatment of periodontal diseases incorporated into the above base, and which is in a gel form for easy applicability before being applied to a periodontal pocket, but has at least a surface portion thereof hardening after application, and resides in the periodontal pocket for a long period, thereby enabling the effect of the active ingredient to persist.

### BRIEF DESCRIPTION OF THE DRAWINGS

FIG. 1 is a graph showing the results of rheometric measurement of bases of the present invention at the start (0 hours of immersion in purified water) of a test for evaluating how the bases of the present invention applied to a topical site changed in hardness (Example 2).

FIG. 2 is a graph showing the results of rheometric measurement of the bases of the present invention immersed in purified water of 37°C for 24 hours in the test for evaluating how the bases of the present invention applied to the topical site changed in hardness (Example 2).

FIG. 3 is a graph showing the results of rheometric measurement of the bases of the present invention immersed in purified water of 37°C for 48 hours in the test for evaluating how the bases of the present invention applied to the topical site changed in hardness (Example 2).

FIG. 4 is a photograph showing the results of investigation of satisfactory residence of the bases of the present invention when the bases were placed in an agar gel (Example 3).

### DETAILED DESCRIPTION OF THE INVENTION

The base of the present invention consists essentially of a water insoluble polymer, and a solvent capable of dissolving the water insoluble polymer and compatible with water, and optionally containing other additives.

This base normally takes a soft gel form, and can be easily applied to a topical site (e.g., a periodontal pocket) as a cream or an ointment by a finger, or through a tube, or by means of a specially designed injection instrument such as a syringe. The solvent compatible with water enables water, which exists around the topical site of application, to enter at least a surface portion of the base in accordance with the outflow of the solvent from the base. As a result of entry of water, the water insoluble polymer hardens at least in the surface portion of the base. The hardened base has elasticity in some cases. Since the base hardens, and in some cases has elasticity, the base retains its shape. Thus, the disadvantage that the applied base, and the active ingredient contained therein are flowed out by a physical force, such as that of water around the topical site, is prevented. Moreover, release of the active ingredient into the tissue can be controlled.

Examples of the water insoluble polymer used in the present invention are one or more polymers selected from the group consisting of aminoalkyl methacrylate copolymer RS, aminoalkyl methacrylate copolymer E, methacrylate copolymer L, methacrylate copolymer S, ethylcellulose, polyvinyl acetal diethylaminoacetate, hydroxypropyl methylcellulose phthalate, hydroxypropyl methylcellulose acetate succinate, carboxymethyl ethylcellulose, cellulose acetophthalate, rosin, sandarac, celluloid, shellac, and zein. However, these polymers are not restrictive, and a water insoluble polymer usable as a component to be added to the active ingredient can be suitably selected and used.

The water insoluble polymer in the base, or its amount incorporated is selected suitably so that when the polymer is dissolved in the solvent and combined with the solvent, a gelled material is formed. In consideration of viscosity and fluidity which affect handling during use or during production, the amount incorporated is typically about 5 to 40% by weight based on the entire composition, or preferably about 10 to 30% by weight if the mode of administration using a syringe is considered.

Examples of the solvent, which dissolves the water insoluble polymer and which is compatible with water, are one or more solvents selected from the group consisting of triethyl citrate, triacetin, tributyrin, diacetylethylene glycol, diethyl sebacate, diethyl phthalate, dibutyl phthalate, ethylene glycol, diethylene glycol, propylene glycol, dipropylene glycol, polyethylene glycol, glycerin, and acetyl glycerin fatty acid ester. However, these solvents are not restrictive, and a solvent usable as a component to be added to the active ingredient can be suitably selected and used.

The solvent in the base, and its amount incorporated are selected suitably so that when the solvent is combined with the water insoluble polymer, a gelled material is formed. In consideration of viscosity and fluidity which affect handling during use or during production, the amount incorporated is typically about 30 to 95% by weight based on the entire composition, or preferably about 40 to 70% by weight if the mode of administration using a syringe is considered.

As the base of the present invention, other additives often used in the pharmaceutical manufacturing field may be incorporated. The type and amount of use of such additive can be determined, as desired, by a person of ordinary skill in the art. Examples of the additive are pH regulators for improving the stability of the active ingredient, and solubility of the active ingredient at the topical site (e.g., organic acids such as adipic acid and fumaric acid, inorganic acid salts such as phosphates, chelating agents such as disodium ethylenediaminetetraacetate); adhesives for improving residence in the tissue where the base is applied (e.g., hydroxypropylcellulose, hydroxypropyl methylcellulose, methylcellulose, polyvinyl alcohol, polyvinylpyrrolidone, sodium carboxymethylcellulose, pullulan, carboxyvinylpolymer, sodium alginate, psyllium gum, gelatin, agar, carrageenan, and dextrin), swelling agents (e.g., low-substituted hydroxypropylcellulose, calcium carboxymethylcellulose, carboxymethylcellulose, crosspovidon, starch, partially pregelatinized starch, and acrylic starch), fats and oils for controlling the rate of hardening of the base and the release of the active ingredient by changing the leachability of the solvent from the base (e.g., paraffin, carnauba wax, rapeseed oil, olive oil, orange oil, eucalyptus oil, peanut oil, wheat germ oil, cacao butter, sesame oil, bleached beeswax, white petrolatum, and microcrystalline wax), fatty acids and their derivatives such as salts and esters (e.g., stearic acid, intermediate chain fatty acid triglyceride, hard fat, calcium stearate, aluminum stearate, and isopropyl myristate), surface active agents (e.g., sucrose ester of fatty acid, lauromacrogol, cetomacrogol, glyceryl monostearate, sorbitan monolaurate, sorbitan monopalmitate, sorbitan trioleate, sorbitan sesquioleate, polyoxyethylene hydrogenated castor oil, and polyoxyl stearate), and polyhydric alcohols/higher alcohols (e.g., polyethylene glycol, propylene glycol, stearyl alcohol, and cetanol). In terms of the physical properties of the preparation and the release of the active ingredient, stearic acid and stearyl alcohol are particularly preferred. When a fat or an oil is incorporated as an additive, that having compatibility with the solvent used is preferred. A fat or oil which is solid at room temperature is dissolved, under heat, in the solvent, and cooled when used.

The amounts of the above-mentioned additives incorporated are suitably set from the aspects of physical properties and releasability required of the preparation or pharmaceutical base.

When the base of the present invention is used to prepare a composition for intraoral administration, an insoluble or slightly soluble additive powder may be incorporated into the solvent in order to improve the properties, such as viscosity and stringiness, before application to a topical site in the mouth. Its examples are polyethylene powder, hydrogenated vegetable oil powder, talc, magnesium stearate, sugar alcohol, anhydrous silicon dioxide, dibasic calcium phosphate, carnauba wax, kaolin, synthetic aluminum silicate, titanium oxide, calcium carbonate, and bentonite. In terms of the physical properties of the preparation and the release of the active ingredient, polyethylene powder, hydrogenated vegetable oil powder, and carnauba wax are particularly preferred.

The additive powder insoluble in the solvent, or its amount incorporated is suitably selected in order to improve handling during production, and the properties, such as viscosity and stringiness, before application to a topical site in the mouth. A typical amount incorporated is about 5 to 50% by weight based on the entire composition. In terms of the dispersion stability in the composition, its specific gravity is preferably in the range of 0.5 to 1.5 relative to the solvent used, and its particle diameter is 100 µm or less, preferably 10 µm or less.

The present invention also provides a pharmaceutical composition or an ointment for topical administration, which is produced by incorporating an active ingredient into the above base, and which takes a gel form or an ointment form before being applied to a topical site, but has a surface portion thereof promptly hardening after application to the topical site, and resides in the topical site for a long period to release the active ingredient slowly in the topical site during this period.

The pharmaceutical composition for topical administration according to the present invention is not restricted in the range of application, and can be used topically in various fields, such as, ophthalmology (ocular mucous membrane), otorhinolaryngology (nasal cavity, oral cavity), dentistry (periodontal disease, dental caries, etc.), oral surgery (preoperative and postoperative measures, etc.), gynecology (vaginal cavity), digestive medicine (rectal cavity, etc.), urology, dermatology, and plastic surgery (wound surface, decubitus surface, etc.). The pharmaceutical composition is also usable as a drug for animals in the same fields.

In the composition of the present invention, the amount of the active ingredient incorporated may be suitably selected in order to obtain the desired effect. The suitable range is about 0.001 to about 30% by weight, more preferably about 0.01 to about 10% by weight, based on the entire base. The active ingredient can be made existent in a state dissolved, dispersed or suspended in the solvent. The active ingredient may be incorporated, as a solution in the selected solvent, into the base, or if not sufficiently soluble in the solvent, may be incorporated, as a fine powder or as a dispersion or suspension in the solvent, into the base. To suppress the rate of release from the base or enhance the stability of the active ingredient, the active ingredient can be incorporated as a fine powder, dispersion or suspension, depending on the method of production or the method of application.

Moreover, if the bulk drug, which is in a crystal or a crystalline powder form, is used, the quality of the resulting composition may be nonuniform as a whole, when the bulk drug is only dispersed in the base directly. In this case, the bulk drug may be ground to a small particle size, or dissolved in a certain solvent, before being kneaded with the base, during the production process.

If the bulk drug is used in ground form, the particle diameter of the active ingredient is not limited. However, it is preferably about 1 µm to about 500 µm, more preferably about 10 µm to about 200 µm, from the aspect of handling during the production process.

The active ingredient may be pretreated before incorporation, if this is necessary to control the rate of release into tissue and improve its stability. Examples of the pretreatment are coating, microcapsulation, adsorption to a porous substance, and adsorption to an ion exchange resin. These techniques are well known in the field of pharmaceutical manufacturing techniques.

The pharmaceutical composition for topical administration according to the present invention can be used as antibiotics, antibacterials, antimycotics, antivirals, bactericides, anti-inflammatories, local anesthetics, vasoconstrictors, steroid hormones, antihistaminics, prostaglandins, or antispasmodics. Active ingredients which can be incorporated into them are not restricted. Examples of the active ingredient include antibiotics, for example, (3-lactams such as ampicillin, aminoglycosides such as kanamycin sulfate, tetracyclines such as tetracycline hydrochloride and minocycline, polypeptides such as polymyxin B sulfate, macrolides such as clarithromycin, and chloramphenicols such as chloramphenicol. As the antibacterial, a new quinolone such as tosufloxacin tosilate, or a synthetic antibacterial such as combination of sulfamethoxazole-trimethoprim is incorporated. As the antimycotic, a polyene macrolide such as amphotericin or an azole and the like is incorporated. As the antiviral, aciclovir or vidarabine is incorporated. As the bactericide, cetylpyridinium chloride or povidone iodine is incorporated. As the anti-inflammatory, glycyrrhizinate dipotassium or lysozyme hydrochloride is incorporated. As the local anesthetic, lidocaine or dibucaine hydrochloride is incorporated. As the vasoconstrictor, naphazoline hydrochloride or dl-methylephedrine hydrochloride is incorporated. As the steroid hormone, hydrocortisone butyrate is incorporated. As the antihistaminic, diphenhydramine hydrochloride or chlorpheniramine maleate is incorporated. As the prostaglandin, dinoprost or dinoprostone is incorporated. As the antispasmodic, crotamiton is incorporated.

Each of the compositions can be used, alone or as a combination of two or more, for treatment or prophylaxis of diseases in various fields, such as ophthalmology, otorhinolaryngology, dentistry, oral surgery, gynecology, digestive medicine, dermatology, urology, and plastic surgery.

An intraoral preparation, in particular, is one of the preferred embodiments of the present invention. Penem compounds are non-natural type β-lactam compounds designed based on the idea of fusing the structures of penicillin and cephalosporin (e.g., Woodward, R.B., In Recent Advances in the Chemistry of β-Lactam Antibiotics; Elks, J., Ed; The Chemical Society; London, 1977; Spec. No. 28, p. 167-180, and Japanese Unexamined Patent Publication Nos. 207387/86, 162694/88, 222486/85, and 119486/79). These compounds are antibiotics of a new type having the broad antibacterial spectrum and high safety of penicillin antibiotics and cephem antibiotics which are β-lactam antibiotics, and the potent antibacterial activity and high stability to β-lactamase of carbapenem antibiotics. Currently, a 2.5hydrate of sodium (+)-(5R,6S)-6-[(R)-1-hydroxyethyl]-7-oxo-3-[(R)-2-tetrahydrofuryl]-4-thia-1-azabicyclo[3.2.0]hept-2-ene-2-carboxylate (faropenem sodim, hereinafter referred to as Compound 1) is used as an oral drug for treatment of various infectious diseases. Because of the novel skeleton, penem ring, this compound is reported to exhibit potent antibacterial activity against Gram-positive bacteria, including not only methicillin sensitive Staphylococcus aureus (MSSA), Streptococcus pyogenes, and Streptococcus pneumoniae, but also penicillin resistant Streptococcus pneumoniae (PRSP), oral streptococci, and enterococci against which conventional β-lactam antibiotics have not been fully effective, and is also reported to show a wide antibacterial spectrum covering Gram-negative bacteria such as Haemophilus influenzae and anaerobic bacteria such as Bacteroides species (Antibiotics & Chemotherapy, Vol. 13, No. 10, p. 74-80, 1997). It is also reported to have potent antibacterial activity against pathogens of periodontitis, including Porphyromonas gingivalis (CHEMOTHERAPY, Vol. 42, S-1, p. 38-50, 1994), as well as species from odontogenic infection which have become noticeably resistant to antibiotics in recent years (Chemotherapy, Vol. 45, No. 11, p. 965-971, 1997).

Generally, penem compounds are chemically very unstable substances against hydrolysis, oxidation or optical isomerization, as are other β-lactam compounds. Thus, it is difficult to produce a pharmaceutical composition for topical administration, the pharmaceutical composition locally utilizing the excellent efficacy of these compounds against inflammatory diseases, pyogenic diseases, and diseases due to infection with resistant strains. However, the base of the present invention, which consists essentially of a water insoluble polymer and a solvent capable of dissolving the water insoluble polymer and compatible with water, has been found to be capable of maintaining a penem compound as suspended or dispersed particles stably for a long period.

The pharmaceutical composition for topical administration according to the present invention is in the form of an ointment or a gel before topical application, and can be administered using a syringe or the like. After administration, this preparation forms a water insoluble film in its surface portion in accordance with leaching of the solvent contained and/or absorption of water. In the meantime, the entire preparation hardens and retains its shape, thus residing at the topical site for a long period. The duration of residence can be designed to last for several weeks. Hence, the composition of the present invention is also suitable for designing a sustained release preparation which slowly releases an active ingredient incorporated to maintain the concentration of the active ingredient at a topical site for a long period.

### Examples

The present invention will now be described more concretely by way of the following examples, which in no way limit the scope of the invention.

### Example 1

The following bases and preparations were prepared. The formulations of the respective compositions are shown in Tables 1, 2, 4, 5 and 7.

### Base Example 1

Aminoalkyl methacrylate copolymer RS (trade name: Eudoragit RS, Röhm, Federal Republic of Germany) was dissolved in triethyl citrate with heating at 90°C, and allowed to cool to room temperature. Then, the mixture was defoamed under reduced pressure to obtain a composition.

### Base Examples 2 to 4

Aminoalkyl methacrylate copolymer RS was dissolved in triethyl citrate with heating at 90°C, and allowed to cool to room temperature. Then, polyethylene powder was added, and uniformly mixed. The resulting mixture was defoamed under reduced pressure to obtain a composition.

### Base Example 5

Aminoalkyl methacrylate copolymer RS was dissolved in triethyl citrate with heating at 90°C, and allowed to cool to room temperature. Then, Food Blue No. 1 was added, and uniformly mixed. The resulting mixture was defoamed under reduced pressure to obtain a composition.

### Base Example 6

Aminoalkyl methacrylate copolymer RS was dissolved in triethyl citrate with heating at 90°C, and allowed to cool to room temperature. Then, Food Blue No. 1 and polyethylene powder were added, and uniformly mixed. The resulting mixture was defoamed under reduced pressure to obtain a composition.

### Base Example 7

Ethylcellulose was dissolved in triethyl citrate with heating at 90°C, and while the solution was hot, stearyl alcohol was added, and mixed uniformly. The mixture was allowed to cool to room temperature, whereafter Food Blue No. 1 was added, and uniformly mixed. The resulting mixture was defoamed under reduced pressure to obtain a composition.

### Comparative Base Example 1

Hydroxyethylcellulose and magnesium chloride (hexahydrate) were dispersed in glycerin, and heated at 100°C until dissolved. After dissolution, the solution was cooled to 50°C to obtain a mixture. Separately, a solution of aminoalkyl methacrylate copolymer RS in triacetin was prepared. This solution was added to the mixture, and the system was uniformly mixed to obtain a composition.

### Comparative Base Example 2

Plastibase, aluminum hydroxide, propylene glycol, and hydroxypropyl methylcellulose were uniformly mixed to obtain a composition.

### Comparative Base Example 3

The formulation of this example contained Comparative Base Example 1 and a suitable amount of Food Blue No. 1 added thereto. That is, hydroxyethylcellulose and magnesium chloride (hexahydrate) were dispersed in glycerin, and heated at 100°C until dissolved. After dissolution, the solution was cooled to 50°C to obtain a mixture. Separately, a solution of aminoalkyl methacrylate copolymer RS in triacetin was prepared. This solution and Food Blue No. 1 were added to the mixture, and the system was uniformly mixed to obtain a composition.

### Comparative Base Example 4

The formulation of this example contained Comparative Base Example 2 and a suitable amount of Food Blue No. 1 added thereto. That is, plastibase, aluminum hydroxide, propylene glycol, hydroxypropyl methylcellulose, and Food Blue No. 1 were uniformly mixed to obtain a composition.

### Preparation Example 1

Aminoalkyl methacrylate copolymer RS was dissolved in triethyl citrate with heating at 90°C, and allowed to cool to room temperature. Then, minocycline hydrochloride and polyethylene powder were added, and the system was uniformly mixed. The resulting mixture was defoamed under reduced pressure to obtain a composition. The minocycline hydrochloride was that which passed through Japanese Pharmacopoeia Standard Sieve No. 100 and remained on JP Standard Sieve No. 200.

### Comparative Preparation Example 1

Hydroxyethylcellulose and magnesium chloride (hexahydrate) were dispersed in glycerin, and heated at 100°C until dissolved. After dissolution, the solution was cooled to 50°C, whereafter minocycline hydrochloride was added and uniformly dissolved to obtain a mixture. Separately, a solution of aminoalkyl methacrylate copolymer RS in triacetin was prepared. This solution was added to the mixture, and the system was uniformly mixed to obtain a composition.

### Preparation Example 2

Aminoalkyl methacrylate copolymer RS was dissolved in triethyl citrate with heating at 90°C, and allowed to cool to room temperature. Then, faropenem sodium and polyethylene powder were added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Preparation Example 3

Ethylcellulose was dissolved in triethyl citrate with heating at 90°C, and allowed to cool to room temperature. Then, faropenem sodium and polyethylene powder were added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Preparation Example 4

Aminoalkyl methacrylate copolymer RS and ethylcellulose were dissolved in triethyl citrate with heating at 90°C, and allowed to cool to room temperature. Then, faropenem sodium and polyethylene powder were added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Preparation Example 5

Aminoalkyl methacrylate copolymer RS was dissolved in triacetin with heating at 90°C, and allowed to cool to room temperature. Then, faropenem sodium and polyethylene powder were added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Preparation Example 6

Ethylcellulose was dissolved in triethyl citrate with heating at 90°C, and while the solution was hot, stearic acid was added, followed by mixing the system uniformly. After the mixture was allowed to cool to room temperature, faropenem sodium was added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Preparation Example 7

Ethylcellulose was dissolved in triethyl citrate with heating at 90°C, and while the solution was hot, stearyl alcohol was added, followed by mixing the system uniformly. After the mixture was allowed to cool to room temperature, faropenem sodium was added, and the system was uniformly mixed. The resulting mixture was defoamed under reduced pressure to obtain a composition. The faropenem sodium was that which passed through JP Standard Sieve No. 100 and remained on JP Standard Sieve No. 200.

### Example 2 Evaluation of shape retention of the preparation (needle penetration test)

To ensure residence at the site of administration, the base of the present invention is characterized by hardening over time in accordance with the outflow of the solvent and entry of water after administration. Changes in the hardness of the base applied topically were evaluated by a rheometer using a model.

Concretely, each of the bases was filled into a cylindrical pocket of 10.0 mm in diameter and 5.0 mm in depth, and allowed to stand in purified water of 37°C. At the start of the test (0 hours), at 24 hours, and at 48 hours, the base was penetrated with a plunger as a needle (needle front end diameter: 4.0 mm) inserted at a rate of 20 mm/min with the use of a rheometer (NRM-3002D, Fudo Kogyo) to measure stress with the passage of time. The formulations of the bases used in the test are shown in Tables 1 and 2.

During penetration at the constant rate, the point of inflection is observed in stress when the base is broken, provided that the base has hardened. The stress at this time of breakage is designated as maximum elastic stress (g), and the distance over which the needle was penetrated is designated as elastic limiting distance (mm). The results are shown in Table 3 and FIGS. 1 to 3.

With Comparative Base Examples 1 and 2, no points of inflection were observed at any of 0, 24 and 48 hours, thus demonstrating that none of these bases hardened. With Base Examples 1, 2, 3 and 4 of the present invention, no points of inflection were observed at 0 hours, but at 24 hours, points of inflection were noted for the needle penetration distance of 1.2 to 2.1 mm. Thus, Base Examples 1, 2, 3 and 4 of the present invention showed the properties of hardening and retaining their shapes during storage in purified water. Also, depending on the amount of the water insoluble polymer incorporated, or the amount of incorporation of the solvent capable of dissolving the water insoluble polymer and compatible with water, the resulting compositions were found to be different in the degree of hardening.

In Base Examples 3 and 4, in particular, the maximum elastic stress and the distance to the point of inflection (elastic limiting distance) at 48 hours were comparable to those at 24 hours. Thus, the present invention was demonstrated to be capable of designing a composition whose properties are kept nearly constant after a lapse of a certain time.

**Table 1**

| Ingredient | Base Ex. 1 | Base Ex. 2 | Base Ex. 3 | Base Ex. 4 |
|---|---|---|---|---|
| Eudoragit RS | 35 | 6 | 13 | 20 |
| Triethyl citrate | 65 | 54 | 51 | 47 |
| Polyethylene powder | | 40 | 36 | 33 |
| Total | 100 | 100 | 100 | 100 |
| (unit: %) | | | | |

**Table 2**

| Ingredient | Comp. Base Ex. 1 | Comp. Base Ex. 2 |
|---|---|---|
| Eudoragit RS | 2.06 | |
| Hydroxyethylcellulose | 4.12 | |
| Magnesium chloride·hexahydrate | 5.15 | |
| Glycerin | 76.30 | |
| Triacetin | 12.37 | |
| Plastibase | | 79.89 |
| Aluminum hydroxide | | 2.76 |
| Propylene glycol | | 2.04 |
| Hydroxypropylmethylcellulose | | 15.31 |
| Total | 100 | 100 |
| (unit: %) | | |

**Table 3**

| Results of Measurement | | | | | | | |
|---|---|---|---|---|---|---|---|
| Time | | Base Ex. of present invention | | | | Comp. Base Ex. | |
| | | 1 | 2 | 3 | 4 | 1 | 2 |
| 0 hrs | Maximum elastic stress (g) | NPI | NPI | NPI | NPI | NPI | NPI |
| | Elastic limiting distance (mm) | | - | - | - | - | - |
| 24 hrs | Maximum elastic stress (g) | 16 | 28 | 33 | 82 | NPI | NPI |
| | Elastic limiting distance (mm) | 1.5 | 1.2 | 1.4 | 2.1 | - | - |
| 48 hrs | Maximum elastic stress (g) | 13 | 15 | 29 | 80 | NPI | NPI |
| | Elastic limiting distance (mm) | 1.4 | 1.3 | 1.2 | 2.0 | - | - |
| NPI: No point of inflection | | | | | | | |

### Example 3 Evaluation of shape retention of base

When applied to a topical site, such as a periodontal pocket, the base of the present invention has at least a surface portion thereof hardening, and can thus maintain its shape, as initially applied, for a long period. To confirm this feature, the following test was conducted:

A 2% agar gel having a slit of 10 mm in width and 20 mm in depth was used as a model of a periodontal pocket. A front end of a plastic piece measuring 5 mm in width, 30 mm in length, and 0.02 mm in thickness was coated with about 10 mg of each preparation, and the plastic piece was inserted into the slit of 10 mm in width and 20 mm in depth provided in the 2% agar gel. The agar gel was stored in an incubator at 37°C, and allowed to stand therein for a certain time. Then, the plastic piece was taken out, and the shape retention of the base incorporating a food color instead of an active ingredient was checked. The formulations of the bases used in the test are shown in Table 4.

As shown in FIG. 4, all of Base Examples 5, 6 and 7 of the present invention nearly retained the initial shape until 48 hours later. On the other hand, Comparative Base Example 3 lost about a half of the initial shape at 6 hours, and lost almost all of the initial shape at 24 hours. Comparative Base Example 4 lost about a half of the initial shape at 1 hour, and lost almost all of the initial shape at 6 hours. Hence, the base of the present invention proved to be excellent in shape retention, and satisfactory in topical residence.

**Table 4**

| Ingredient | Base Ex. 5 | Base Ex. 6 | Base Ex. 7 | Comp. Base Ex. 3 | Comp. Base Ex. 4 |
|---|---|---|---|---|---|
| Food Blue No. 1 | 0.20 | 0.20 | 0.20 | 0.20 | 0.20 |
| Ethylcellulose | - | - | 16.29 | - | - |
| Eudoragit RS | 34.93 | 26.48 | - | 2.06 | - |
| Triethyl citrate | 64.87 | 49.90 | 63.14 | - | - |
| Polyethylene powder | - | 23.42 | - | - | - |
| Stearyl alcohol | - | - | 20.37 | - | - |
| Hydroxyethylcellulose | - | | - | 4.12 | - |
| Magnesium chloride hexahydrate | - | - | - | 5.14 | - |
| Triacetin | - | - | - | 12.35 | - |
| Glycerin | - | - | - | 76.13 | - |
| Plastibase | - | - | - | - | 79.73 |
| Aluminum hydroxide | - | - | - | - | 2.75 |
| Propylene glycol | - | - | - | - | 2.04 |
| Hydroxypropyl methylcellulose | - | - | - | - | 15.28 |
| Total | 100.00 | 100.00 | 100.00 | 100.00 | 100.00 |
| (unit: %) | | | | | |

### Example 4 Confirmation (in vitro) of drug release rate

The rate of drug release from the preparation of each of Preparation Examples 1 to 7 was confirmed in vitro. To examine the release rate of the drug at the site of administration, a test was conducted using a model of a periodontal pocket. Concretely, the composition filled into a syringe having a front end internal diameter of 300 µm was accurately weighed in an amount of about 10 mg, and coated in a nearly circular form onto a front end of a plastic piece measuring 5 mm in width, 30 mm in length, and 0.02 mm in thickness. The plastic piece was inserted into a slit of 10 mm in width and 20 mm in depth provided in a 2% agar gel. The agar gel was stored in an incubator at 37°C, and the plastic piece was taken out after a lapse of a certain time. Then, the drug remaining in the preparation was quantitatively determined.

Conditions for analysis of faropenem sodium: A high performance liquid chromatographic column of stainless steel packed with octadecylsilylated silica gel was used. The column temperature was set at 40°C. As a mobile phase, a mixture of water, acetonitrile and a 1 mol/l aqueous solution of phosphoric acid (200 ml:900 ml:10 ml) incorporating 3.1 g of sodium dihydrogenphosphate was used. The flow rate was adjusted so that the retention time of faropenem sodium would be about 14 minutes. An ultraviolet absorption spectrophotometer was used as a detector, and a measurement wavelength of 305 nm was used.

Conditions for analysis of minocycline hydrochloride: A high performance liquid chromatographic column of stainless steel packed with octadecylsilylated silica gel was used. The column temperature was set at 40°C. The mobile phase used was a solution of 20.2 g of triethylamine in a mixture of water and acetonitrile (100 ml:50 ml), the solution being adjusted to pH 2.2 with the addition of phosphoric acid. The flow rate was adjusted so that the retention time of minocycline hydrochloride would be about 14 minutes. An ultraviolet absorption spectrophotometer was used as a detector, and a measurement wavelength of 354 nm was used.

The formulations of the preparations used in the analyses are shown in Tables 5 and 7. The corresponding results of analyses are shown in Tables 6 and 8.

A comparison between Preparation Example 1 and Comparative Preparation Example 1 using minocycline hydrochloride as the active ingredient showed that Preparation Example 1 with satisfactory shape retention of the preparation had a large residual amount of the active ingredient residing at the topical site of administration. The results on Preparation Examples 2 to 7 using faropenem sodium as the active ingredient showed that the release rate of the active ingredient could be controlled arbitrarily, depending on the differences in the types and/or amounts of incorporation of the water insoluble polymer, the solvent capable of dissolving the water insoluble polymer and compatible with water, and other additives.

**Table 5**

| Formulations of Preparation Example 1 and Comparative Preparation Example 1 | | |
|---|---|---|
| Ingredient | Preparation Ex. 1 | Comp. Preparation Ex. 1 |
| Minocycline hydrochloride | 2 | 2 |
| Aminoalkyl methacrylate copolymer RS | 26 | 2 |
| Triethyl citrate | 49 | |
| Polyethylene powder | 23 | |
| Hydroxyethylcellulose | | 4 |
| Magnesium chloride.hexahydrate | | 5 |
| Glycerin | | 75 |
| Triacetin | | 12 |
| Total | 100 | 100 |
| (unit: %) | | |

**Table 6**

| Residual Amount (%) of Minocycline Hydrochloride in Preparation | | |
|---|---|---|
| Time | Preparation Ex. 1 | Comp. Preparation Ex. 1 |
| 2 hr | 94.3 | 47.1 |
| 18 hr | 28.8 | 4.4 |

**Table 7**

| Formulations of Preparation Examples 2 to 7 | | | | | | |
|---|---|---|---|---|---|---|
| Ingredient | Pre. Ex.2 | Pre. Ex.3 | Pre. Ex.4 | Pre. Ex.5 | Pre. Ex.6 | Pre. Ex.7 |
| Faropenem sodium | 2 | 2 | 2 | 2 | 2 | 2 |
| Ethylcellulose | | 14 | 7 | | 16 | 16 |
| Aminoalkyl methacrylate copolymer RS | 26 | | 13 | 26 | | |
| Triethyl citrate | 49 | 54 | 52 | | 62 | 62 |
| Triacetin | | | | 49 | | |
| Polyethylene powder | 23 | 30 | 26 | 23 | | |
| Stearic acid | | | | | 20 | |
| Stearyl alcohol | | | | | | 20 |
| Total | 100 | 100 | 100 | 100 | 100 | 100 |
| (unit: %) | | | | | | |

**Table 8**

| Residual Amount (%) of Faropenem Sodium in Preparation | | | | | | |
|---|---|---|---|---|---|---|
| Time | Prep. Ex.2 | Prep. Ex.3 | Prep. Ex.4 | Prep. Ex.5 | Prep. Ex.6 | Prep. Ex.7 |
| 2 hr | 48.9 | 97.2 | 59.2 | 34.2 | 89.8 | 91.7 |
| 18 hr | 6.9 | 9.3 | 8.1 | 5.5 | 26.5 | 43.3 |

## Claims

1. A pharmaceutical composition for topical administration, comprising an active ingredient, a water insoluble polymer, and a solvent capable of dissolving the water insoluble polymer and compatible with water, and optionally containing other additive.

2. The pharmaceutical composition for topical administration as claimed in claim 1, which is an antibiotic, an antibacterial, an antimycotic, an antiviral, a bactericide, an anti-inflammatory, a local anesthetic, a vasoconstrictor, a steroid hormone, an antihistaminic, a prostaglandin, or an antispasmodic in an animal including human.

3. The pharmaceutical composition for topical administration as claimed in any one of claims 1 to 2, which is used for intraoral administration.

4. The pharmaceutical composition for topical administration as claimed in any one of claims 1 to 3, wherein the water insoluble polymer is one or more polymers selected from the group consisting of aminoalkyl methacrylate copolymer RS, aminoalkyl methacrylate copolymer E, methacrylate copolymer L, methacrylate copolymer S, ethylcellulose, polyvinyl acetal diethylaminoacetate, hydroxypropyl methylcellulose phthalate, hydroxypropyl methylcellulose acetate succinate, carboxymethyl ethylcellulose, cellulose acetophthalate, rosin, sandarac, celluloid, shellac, and zein.

5. The pharmaceutical composition for topical administration as claimed in any one of claims 1 to 4, wherein the solvent capable of dissolving the water insoluble polymer and compatible with water is one or more solvents selected from the group consisting of triethyl citrate, triacetin, tributyrin, diacetylethylene glycol, diethyl sebacate, diethyl phthalate, dibutyl phthalate, ethylene glycol, diethylene glycol, propylene glycol, dipropylene glycol, polyethylene glycol, glycerin, and acetyl glycerin fatty acid ester.

6. The pharmaceutical composition for topical administration as claimed in any one of claims 1 to 5, wherein an additive powder insoluble or slightly soluble in the solvent is incorporated as the other additive.

7. The pharmaceutical composition for topical administration as claimed in any one of claims 1 to 5, wherein a pH adjustor is incorporated as the other additive.

8. The pharmaceutical composition for topical administration as claimed in any one of claims 1 to 5, wherein an adhesive and/or a swelling agent are or is incorporated as the other additive.

9. The pharmaceutical composition for topical administration as claimed in any one of claims 1 to 5, wherein one or more additives selected from the group consisting of fats and oils, fatty acids and fatty acid derivatives such as salts or esters of the fatty acids, surface active agents, polyhydric alcohols, and higher alcohols are incorporated as the other additives.

10. The pharmaceutical composition for topical administration as claimed in any one of claims 1 to 9, which is a sustained release preparation.

11. The pharmaceutical composition for topical administration as claimed in claim 10, wherein the active ingredient is a penem antibiotic.

12. A pharmaceutical base comprising a water insoluble polymer, and a solvent capable of dissolving the water insoluble polymer and compatible with water, and optionally containing additive.

13. The pharmaceutical base of claim 12, which is used in a pharmaceutical composition for topical administration.

14. Use of a water insoluble polymer for production of a pharmaceutical composition for topical administration by mixing with a solvent capable of dissolving the water insoluble polymer and compatible with water.

15. Use of a solvent for production of a pharmaceutical composition for topical administration by mixing with a water insoluble polymer, the solvent capable of dissolving the water insoluble polymer and compatible with water.

16. An agent for treatment of periodontal disease, comprising an active ingredient for treatment of periodontal disease, a water insoluble polymer, and a solvent capable of dissolving the water insoluble polymer and compatible with water, and optionally other additive.
